# FASCICULE DE BREVET EUROPEEN

(11) **EP 0 593 506 B1**
(45) Date de publication et mention de la délivrance du brevet: **27.03.1996**
(21) Numéro de dépôt: 92911537.6
(22) Date de dépôt: 21.05.1992
(51) Int. Cl.: C07C 229/12, A61K 7/40, A61K 31/19

(54) **LIPOAMINOACIDES, LEUR PROCEDE DE PREPARATION ET LEURS APPLICATIONS**
LIPOAMINOSAUREN,IHRE HERSTELLUNG UND VERWENDUNG
LIPOAMINOACIDS, PREPARATION METHOD AND APPLICATIONS

(30) Priorité: 22.05.1991 FR 9106143
(43) Date de publication de la demande: 27.04.1994
(73) Titulaire: GIVAUDAN-LAVIROTTE, F-69008 Lyon (FR)
(72) Inventeur: MORELLE, Jean, F-75010 Paris (FR); LAUZANNE-MORELLE, Eliane, F-75010 Paris (FR); ROTHFUSS-MORELLE, Jacqueline, F-67630 Lauterbourg (FR)
(74) Mandataire: Caen, Thierry Alain
(86) Numéro de dépôt international: FR9200450
(87) Numéro de publication internationale: WO9220647

(56) Documents cités:
- EP-A- 0 126 009
- DD-A- 240 125
- FR-A- 2 403 024
- FR-A- 2 422 400
- CHEMICAL ABSTRACTS, vol. 108, 1988, Columbus, Ohio, US; abstract no. 11005, BATTIONI ET AL: "Lipoamino acids in cleansing products" page 316; colonne 1;
- CHEMICAL ABSTRACTS, vol. 112, 1990, Columbus, Ohio, US; abstract no. 104555, BRAUN, D.B.: 'Developments with lipoamino acids and their salts' page 372 ;colonne 1 ;
- JOURNAL DE PHARMACIE DE BELGIQUE vol. 46, no. 4, 1991, BRUXELLES pages 266 - 270; TRANIELLO ET AL: 'Biologie cellulaire et cosmétologie'

## Description

La présente invention concerne des lipopolyaminoacides consistant en des mélanges d'acides aminés N-acylés, lesdits acides aminés étant obtenus par hydrolyse totale de protéines, leur procédé de préparation et leurs applications, notamment dans des compositions cosmétiques, pharmaceutiques, détergentes ou alimentaires.

Les lipopolyaminoacides consistant en des mélanges d'acides aminés et/ou de peptides obtenus par hydrolyse totale ou partielle de protéines, N-acylés par des radicaux acyles, sont bien connus et sont utilisés dans de nombreux domaines d'application.

Ainsi la demande de brevet français n° 2.403.024 décrit l'utilisation dans le domaine agricole, notamment en tant que fertilisant, des sels métalliques, de peptides ou d'acides aminés acylés. Les peptides et acides aminés sont obtenus par hydrolyse partielle ou totale de protéines animales, essentiellement la kératine et le collagène. Ces derniers constituent à côté de l'élastine et de la caséine, les principales sources de protéines animales mises en oeuvre en vue de l'obtention de lipopolyaminoacides.

Ces lipopolyaminoacides consistant en des mélanges d'acides aminés acylés, ou de peptides acylés, issus d'hydrolysats de protéines animales, ont cependant pour inconvénients de présenter des risques potentiels de contamination par des agents pathogènes, tels des virus, occasionnellement présents dans les tissus animaux.

Ces risques potentiels conduisent de plus en plus fréquemment les utilisateurs à se détourner de tout composé issu de l'animal.

On connait par ailleurs des protéines acylées d'origine végétale.

L'article de Zahurul Haque et al., "Incorporation of fatty acid into food protein : palmitoyl soybean glycinin", J. Agric. Food. Chem. 1982, 30, 481-486, décrit une protéine de soja acylée par un radical palmitoyle et présentant une activité émulsifiante et une stabilité de mousse améliorée par rapport à la protéine non acylée.

Toutefois, il a pu être constaté que de telles protéines acylées ont pour inconvénient de présenter un faible pouvoir mouillant et une mousse encore insuffisamment stable notamment en eau dure ou en présence de salissures.

La présente invention a alors pour objet des lipopolyaminoacides ne comportant pas de risques potentiels de contamination de l'homme ou de l'animal par des agents pathogènes.

Par ailleurs les lipopolyaminoacides de l'invention permettent de conférer un pouvoir mouillant important ainsi que l'obtention d'une mousse stable même en eau dure ou en présence de salissures.

La présente invention concerne donc des lipopolyaminoacides consistant en un mélange d'acides aminés obtenu par hydrolyse totale d'une protéine, lesdits acides aminés étant, N-acylés par un radical acyle en C₄ - C₃₀, caractérisé en ce que la protéine est issue d'un tourteau d'une plante oléagineuse.

Dans le cadre de la présente invention, on entend par tourteau la masse de poudre grossière formée avec le résidu de certaines plantes ou de parties de ces plantes, tels leurs graines, et dont on a exprimé l'huile. De tels tourteaux sont habituellement destinés à la nourriture des animaux ou utilisés comme engrais. Ces tourteaux peuvent comporter jusqu'à 50% en poids, voire plus, de protéines et constituent donc une source protéique particulièrement riche.

Lesdites protéines peuvent être hydrolysées alors qu'elles sont encore contenues dans le tourteau, ou préalablement à leur hydrolyse, être séparées dudit tourteau par des méthodes classiques. Dans ce dernier cas, ces protéines peuvent alors notamment se présenter sous la forme d'un concentrat dont la teneur en protéines peut atteindre jusqu'à 70 % en poids, sous la forme d'un isolat dont la teneur en protéines peut atteindre 90 % en poids ou sous la forme d'une protéine purifiée.

Parmi les plantes oléagineuses à partir desquelles sont préparées lesdits tourteaux, on peut citer celles appartenant à la famille des crucifères, tel le colza, à la famille des oléacés, tel l'olivier ou à la famille des légumineuses tels le lupin, l'arachide, ou, plus avantageusement le soja.

Les lipopolyaminoacides de l'invention sont constitués principalement de mélanges d'acides aminés N-acylés, également connus sous le terme "lipoaminoacides", de formule générale (I): 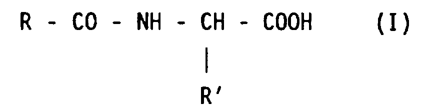
où R représente un radical aliphatique en C₃ - C₂₉, de préférence en C₇ - C₁₉ saturé ou insaturé, linéaire ou ramifié,
et R' représente la chaine principale d'un acide aminé issu de l'hydrolyse totale d'une protéine issue d'un tourteau d'une plante oléagineuse .

Selon un aspect avantageux de l'invention, le radical acyle est choisi parmi les radicaux octanoyle, undécylénoyle, lauroyle, cocoyle, palmitoyle, stéaroyle, linoléoyle ou oléoyle.

La fonction carboxylique des lipoaminoacides de formule I peut être libre ou salifiée. Dans ce dernier cas, les lipopolyaminoacides de l'invention peuvent être salifiés par l'ammoniaque, des cations de métaux alcalins, comme le sodium ou le potassium, ou alcalino-terreux, comme le calcium ou le magnésium, ou des cations de métaux tels le cobalt, le fer, le manganèse, le cuivre, le zinc, l'aluminium, notamment sous la forme de sels monobasique ou dibasique d'aluminium. Ils peuvent aussi être salifiés par de bases organiques telles les mono, di ou triethanolamine, la lysine, l'arginine, l'histidine, l'ornithine, la choline ou la morpholine.

L'invention concerne également un procédé de préparation des lipopolyaminoacides décrits ci-dessus.

Selon ce procédé, on hydrolyse totalement une protéine issue d'un tourteau d'une plante oléagineuse en milieu acide de sorte à obtenir un mélange d'acides aminés, on amène le pH de ce mélange à une valeur comprise entre 8 et 11 au moyen d'une base, puis on N-acyle lesdits acides aminés selon une méthode classique, et, le cas échéant, on fait réagir les acides aminés N-acylés avec une base minérale ou organique en vue de former un sel correspondant.

Par hydrolyse totale, on signifie dans le cadre de la présente invention, qu'on libère substantiellement tous les acides aminés constitutifs de la protéine. Une telle hydrolyse totale peut être confirmée par une absence de réaction biurétique. L'hydrolyse totale peut cependant conduire selon les conditions de mise en oeuvre, à un mélange d'acides aminés libres comportant une faible proportion d'oligopeptides ; la longueur de chaine moyenne de ce mélange étant d'environ 1,4 ou moins. Généralement, la proportion de ces oligopeptides dans ledit mélange est comprise entre 1 et 5 % en poids. Les lipopolyaminoacides obtenus à partir d'un mélange comportant des acides aminés libres et des oligopeptides, font partie intégrante de la présente invention.

En vue d'obtenir uniquement des acides aminés libres, on peut, le cas échéant, procéder à une opération de séparation des oligopeptides dudit mélange, mise en oeuvre de manière classique. Une telle opération classique peut par exemple consister en une séparation sur résine échangeuse d'ions.

L'hydrolyse totale peut être effectuée en solution aqueuse au moyen d'un acide fort, tel l'acide chlorhydrique, notamment en une concentration de l'ordre de 25 % à 35% en poids. La température à laquelle est réalisée l'hydrolyse est habituellement comprise entre 60 et 130°C.

Suite à l'hydrolyse, le pH du mélange en résultant peut être ramené à un pH compris entre 3 et 7, plus généralement voisin de 5, au moyen d'une base, telle la soude, en vue d'une opération de décoloration et de filtration, effectuée classiquement.

Lorsque l'hydrolyse totale est effectuée sur une protéine en mélange avec d'autres composés, notamment lorsque l'hydrolyse est effectuée directement sur une protéine non séparée du tourteau, le mélange en résultant est généralement filtré, en particulier en vue d'éliminer les hydrates de carbone constitutif dudit tourteau.

La N-acylation des acides aminés, et le cas échéant des oligopeptides, contenus dans le mélange résultant de l'hydrolyse totale, peut être effectuée au moyen de dérivés d'acides carboxyliques en C₄ - C₃₀ activés.

De tel dérivés activés sont, de préférence, l'anhydride symétrique de ces acides ou des chlorures d'acides. Cette opération s'effectue généralement à une température comprise entre 0° et 100°C.

Les lipopolyaminoacides ainsi obtenus peuvent être éventuellement purifiés ou décolorés par des méthodes classiques telles que la cristallisation ou la chromatographie.

Des sels de lipopolyaminoacides ainsi préparés peuvent alors être obtenus par réaction avec des bases organiques ou minérales, ou avec des dérivés métalliques. A titre de bases organiques ou minérales, on peut citer la potasse, la soude, la chaux, la magnésie, l'ammoniaque, la monoéthanolamine, la diéthanolamine, la triéthanolamine, la morpholine, l'histidine, l'ornithine, la lysine, l'arginine ou la choline.

Les lipopolyaminoacides de l'invention peuvent être utilisés dans de nombreux domaines d'application.

Ils peuvent notamment être mis en oeuvre dans des compositions cosmétiques ou alimentaires dans lequels ils sont généralement présents à des teneurs comprises entre 0,5 et 20 % en poids, plus généralement entre 1 et 10 % en poids.

De tellles compositions cosmétiques peuvent être notamment des shampooings, des savons, des crèmes, des laits, des mousses, des aérosols, des gels, des sticks, des huiles, des émulsions, des pâtes dentifrices ou des lotions aqueuses ou alcooliques.

Dans ces compositions cosmétiques, les lipopolyaminoacides de l'invention sont notamment utilies en tant qu'émollients, tensioactifs, et/ou régulateurs du pH de la peau. Les propriétés d'émollients et de tensioactifs peuvent bien entendu être utilisées dans d'autres applications.

Ces lipopolyaminoacides peuvent être utilisés dans des compositions détergentes auxquelles ils confèrent un fort pouvoir moussant, nettoyant et épaississant.

Par composition détergente, on signifie aussi bien des compositions destinées à l'hygiène corporelle, notamment des cheveux et de la peau que celles plus particulièrement destinées au lavage, au nettoyage ou à l'entretien des sols et surfaces, de la vaisselle et surtout des textiles comme les textiles délicats, en laine, soie, lin ou coton.

Pour leurs utilisations dans des compositions détergentes plus particulièrement destinées à l'hygiène corporelle, on met avantageusement en oeuvre des lipopolyaminoacides N-acylés par un radical lauroyle ou cocoyle, sous forme de sels alcalins. Le pH de telles compositions détergentes se situe avantageusement entre 6 et 7.

Ces compositions détergentes contiennent habituellement de 10 à 30 % en poids de lipopolyaminoacides selon l'invention.

L'intérêt des lipopolyaminoacides selon l'invention dans des compositions détergentes, a pu être mis en évidence en comparant leur pouvoir moussant à celui de lipopolyaminoacides obtenus à partir de protéines animales. Ainsi on a pu constater qu'une solution aqueuse ne comportant que 20% en poids d'un sel de sodium d'un lipopolyaminoacide, résultant de l'hydrolyse totale d'une protéine de soja et acylés par un radical lauroyle, permet l'obtention d'une mousse de qualité identique à celle obtenue avec une solution aqueuse comportant 36% en poids d'un sel de sodium d'un lipopolyaminoacide, résultant d'une hydrolyse totale du collagène et acylés par un radical lauroyle.

Les lipopolyaminoacides de l'invention, notamment ceux sous forme de sel de cobalt, fer, manganèse, cuivre ou zinc peuvent être utilisés comme médicament, destiné à l'homme, l'animal ou aux plantes, en particulier comme médicament anti-inflammatoire et antimicrobien de par leur propriétés antibactériennes et antifongiques. Parmi les lipopolyaminoacides les plus efficaces comme antimicrobiens figurent ceux N-acylés par un radical octanoyle ou undécylénoyle.

Les sels d'aluminium monobasique et dibasique des lipopolyaminoacides selon l'invention, notamment ceux N-acylés par un radical undécylénoyle, sont encore utiles en tant qu'agent antisudoral pour lutter contre l'hyperhydrose.

Les lipopolyaminoacides de la présente invention peuvent aussi constituer des matières actives dans des compositions destinées à l'agriculture, notamment en tant qu'agent de stimulation de la croissance des plantes et/ou agent de protection contre les parasites, les bactéries et les champignons.

Enfin, il a pu être constaté que les lipopolyaminoacides de l'invention, obtenus à partir de protéines issues de tourteaux de plantes oléagineuses présentaient des propriétés anti-radicalaires notables, en particulier ceux N-acylés par un radical oléique.

Ils peuvent donc être utilisés comme agents anti-radicalaires.

Les exemples suivants on pour but d'illustrer la présente invention:

### Exemple 1

Préparation du sel de sodium du lauroylpolyaminoacide de soja (sel de sodium d'un mélange d'acides aminés, N-lauroylés, obtenu par hydrolyse totale d'une protéine d'un tourteau de soja).

Un mélange constitué de 300g de tourteau de soja, de 300ml d'acide chlorhydrique à 30% et de 100ml d'eau est porté à reflux pendant 6 heures. Après refroidissement, le pH de la solution ainsi obtenue est amené à 4 au moyen d'une solution de soude. on filtre la solution pour éliminer les hydrates de carbone, puis on ramène le pH aux environs de 2,5 à l'aide d'une solution d'acide chlorhydrique à 30%. on traite la solution par 10g de noir décolorant sous agitation pendant une heure à 60°C. Après refroidissement, on filtre la solution pour éliminer le noir décolorant. On obtient une solution comportant des acides aminés et des oligopeptides ; la longueur de chaine moyenne étant de 1,3 (mesurée par le rapport azote total/azote aminé).

On ajuste le pH de la solution décolorée à 10 au moyen de soude puis on y introduit 155g de chlorure de lauroyle, tout en maintenant le pH de la solution à 10 au moyen de soude, sans dépasser une température de 40°C.

On porte la solution à 70°C pendant une heure, on laisse refroidir jusqu'à 30°C et on acidifie à pH 1,5 au moyen d'une solution d'acide chlorhydrique.

La phase aqueuse inférieure est éliminée, et la phase surnageante est lavée par de l'eau à 70°C, jusqu'à l'élimination de l'acide chlorydrique. On ajoute 600 g d'eau et on neutralise à pH7 au moyen d'une solution aqueuse de soude.

On obtient ainsi 780g d'une solution contenant environ 150g de lipolyaminoacide consistant en un sel de sodium d'un mélange d'acides aminés N-lauroylés obtenu par hydrolyse totale d'une protéine de soja, soit une solution à 19,2% en poids dudit lipopolyaminoacide.

### Exemple 2

### Préparation du sel de sodium du lauroylpolyaminoacide de soja

Un mélange constitué de 340 g de protéine de soja, 225 cm³ d'eau et 450 cm³ d'acide chlorhydrique à 30% en poids/poids est porté pendant 6 heures à reflux. Après refroidissement, 225 cm³ d'eau sont ajoutés à la masse réactionnelle qui est ensuite ajustée à pH 3.5 avec 380 cm³ de soude à 30% poids/poids. On ajoute alors 20 g de noir décolorant, on laisse une heure en contact sous agitation, puis on filtre la suspension.

La solution obtenue est ensuite portée à pH 10.5 à l'aide de 260 cm³ de soude à 30% poids/poids, puis chauffée à 40 - 45°C sous vide (7,99 10 Pa) pendant 4 heures, et traitée à nouveau à l'aide de 20 g de noir décolorant pendant 20 minutes sous agitation. Après filtration, on obtient 1641 g d'hydrolysat de protéine de soja.

A 500g de cet hydrolysat, on ajoute 120 cm³ d'eau, on porte la température à 30°C puis on coule simultanément, sous agitation, 102g de chlorure de lauroyle et 60 cm³ de soude 30 % poids/poids en maintenant le pH à 10.5 ± 0.3 et la température au dessous de 40°C, en heure environ. Après la fin d'addition, la température est maintenue à 40°C pendant 30 minutes, puis à 60°C pendant 1 heure. On élève alors la température à 85°C puis on ajoute 130 cm³ d'acide chlorhydrique 30% poids/poids, de manière à porter le pH vers 1. L'agitation est alors arrêtée et la phase aqueuse inférieure est soutirée. A la phase organique, on ajoute, sous agitation, 400 cm³ d'eau et on porte le mélange à pH 7 avec 46 cm³ de soude 30% poids/poids. La solution est traitée à 80°C avec 10 g de noire décolorant, puis filtrée et l'on obtient ainsi 480 g d'une solution aqueuse à 30% de sel de sodium du dérivé N-lauroylé des amino acides obtenues par hydrolyse totale de la protéine de soja, sous forme d'une solution limpide, visqueuse, jaune à brun clair.

### Exemple 3

### Préparation du palmitoylpolyaminoacide de soja

A 600 g d'hydrolysat de protéine de soja à pH 10.5 obtenu de la manière décrite à l'essai précécent, 120 cm³ d'eau et 85 cm³ d'isopropanol sont ajoutés, la température est portée à 30°C puis on ajoute simultanément, sous agitation, 135g de chlorure de palmitoyle et 65 cm³ de soude à 30 % poids/poids en maintenant le pH à 10,5 ± 0,3 et la température en dessous de 40°C pendant 30 minutes, puis à 60°C pendant une heure, on porte ensuite à 80°C avant d'ajouter 135 cm³ d'acide chlorhydrique 30% poids/poids de façon à porter le pH vers 1. L'agitation est alors arrêtée, la phase aquese inférieure est soutirée. La phase organique est lavée trois fois, à 90°C, par 250 cm³ d'une solution aqueuse à 10% de chlorure de sodium. Après le dernier lavage, la phase organique, maintenue à 90°C est séchée, sous agitation, sous vide de 7,99 10 Pa, puis coulée et refroidie. On obtient ainsi 171 g du dérivé N-palmitoylé des amino acides obtenus par hydrolyse totale de la protéine de soja sous forme d'une cire beige clair.

### Exemple 4

Le pouvoir mouillant de lipopolyaminoacides selon l'invention a été mesuré selon le test AFNOR NFT 73406 sur des solutions aqueuses à 0,1 % en poids de matière active.

Selon ce test, le pouvoir mouillant est inversement proportionnel au temps (en s) mesuré.

Les lipopolyaminoacides testés sont les suivants :
- sel de sodium du lauroylpolyaminoacide de soja de l'exemple 1 (A)
- sel de sodium du cocoylpolyaminoacide de soja (B)
   et à titre comparatif :
- sel de triéthanolamine du cocoylpolyaminoacide de collagène (c)
- sel de sodium du cocoylpolyaminoacide de collagène (D)

Les résultats obtenus figurent dans le Tableau I ci-dessous.

**TABLEAU I**

| Matière active | Temps mesuré (s) |
|---|---|
| A | 28 |
| B | 43 |
| C | 230 |
| D | 100 |

### Exemple 5

Les tableaux II et III ci-dessous concernent les dosages d'azote et les mesures d'indices d'acides de lipopolyaminoacides selon l'invention obtenus à partir de tourteaux d'origine diverses.

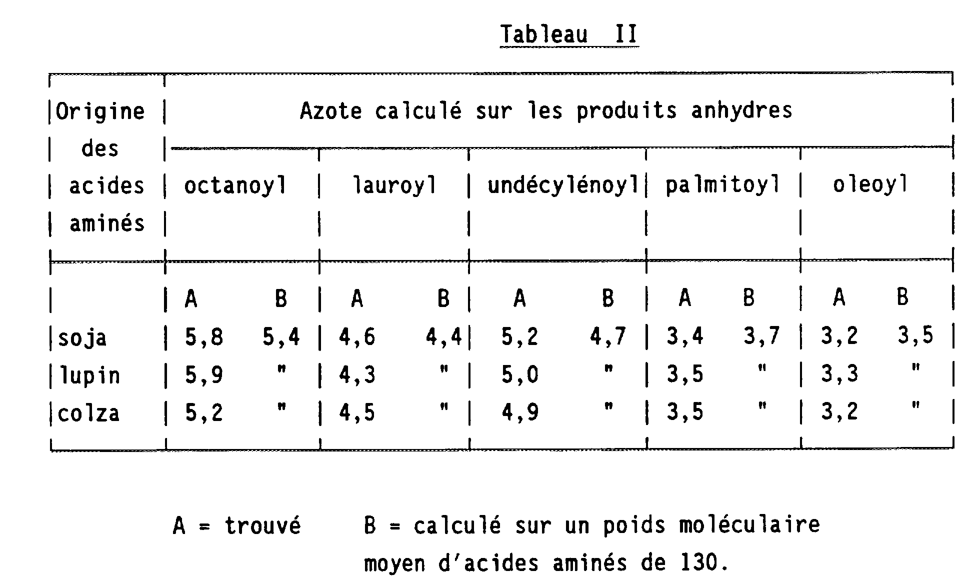

Ces chiffres peuvent varier de 5 à 10 % du fait de la présence d'acides gras libres et des rapports des aminoacides dans les protéines végétales.

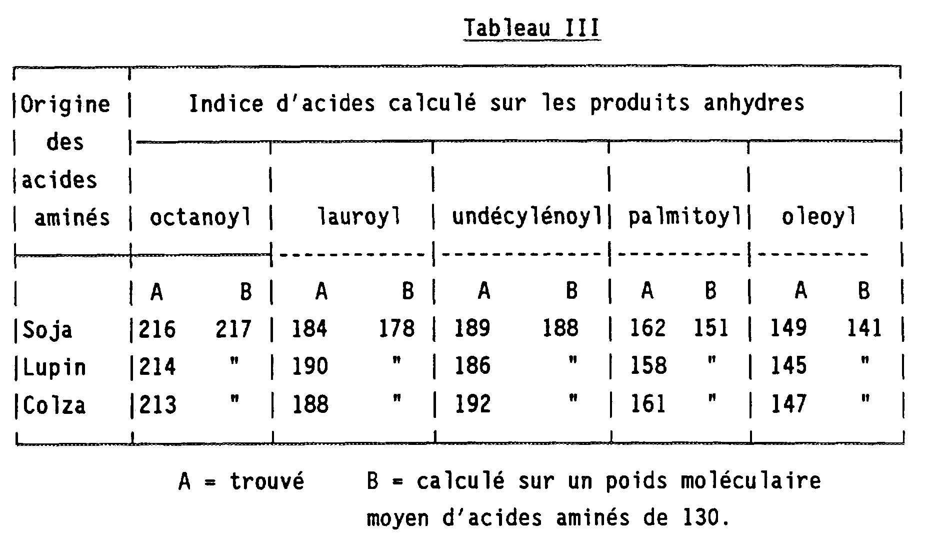

### Exemples 6 à 13

Ces exemples sont relatifs à des compositions contenant des lipopolyaminoacides selon l'invention. Elles sont toutes obtenues par simple mélange à chaud ( 60°C environ) de leurs différents constituants. Les teneurs en ces constituants sont exprimées en g.

### Exemple 6

| Composition pour l'entretien physiologique de la peau. | |
|---|---|
| | |
| acide stéarique | 10 |
| alcool cétylique polyoxyéthylèné (20) | 5 |
| Lypopolyaminoacides issus de protéines de soja, N-acylés par un radical oléique (oléoylpolyaminoacides de soja) | 3 |
| glycérine | 10 |
| eau | qs100 |

### Exemple 7

| composition antiinflammatoire | |
|---|---|
| | |
| acide stéarique | 10 |
| stéarate de polyoxyéthyléné (100) | 5 |
| alcool cétylique | 2 |
| palmitoylpolyaminoacides de soja | 10 |
| glycérol | 10 |
| Eau | qs100 |

### Exemple 8

| Composition antifongique | |
|---|---|
| | |
| alcool gras polyoxyéthyléné (20) | 5 |
| octanoylpolyaminoacides de soja | 2 |
| undécylénoylpolyaminoacides de colza | 2 |
| eau | qs100 |

### Exemple 9

| emulsion antitranspirante | |
|---|---|
| | |
| stéarate polyoxyéthyléné (100) | 10 |
| alcool cétylique polyoxyéthyléné (20) | 5 |
| propylénéglycol | 10 |
| sel d'aluminium monobasique d'undécylénoyl polyaminoacides de soja | 5 |
| eau | qs100 |

### Exemple 10

| shampooing | |
|---|---|
| | |
| Sel d'ammonium de lauroylpolyaminoacides de soja (à 20 % en poids de matière active) | 30 |
| octanoylpolyaminoacides de soja | 0,5 |
| eau | qs100 |

### Exemple 11

| savon | |
|---|---|
| | |
| savon dit de toilette | 96 |
| sel de sodium de lauroylpolyaminoacides de colza (à 20 % en poids de matière active) | 4 |

### Exemple 12

| composition antifongique pour l'agriculture | |
|---|---|
| | |
| sel de cuivre de l'octanoyl polyaminoacides de soja (à 20 % en poids de matière active) | 5 |
| ammoniaque à 30 % | 5 |
| eau | qs100 |

### Exemple 13

| composition pour l'alimentation animale | |
|---|---|
| | |
| sel de cobalt de palmitoylpolyaminoacides de soja (à 20 % en poids de matière active) | 1 |
| sel de manganèse | 1 |
| sel de cuivre | 1 |
| poudre de tourteau de soja | 97 |

## Revendications

1. Lipopolyaminoacides consistant en un mélange d'acides aminés obtenu par hydrolyse totale d'une protéine, lesdits acides aminés étant N-acylés par un radical acyle en C₄ - C₃₀, caractérisé en ce que la protéine est issue d'un tourteau d'une plante oléagineuse.

2. Lipopolyaminoacides selon la revendication 1 caractérisés en ce que ladite protéine issue d'un tourteau se présente sous la forme d'un isolat, d'un concentrat ou d'une protéine purifiée.

3. Lipopolyaminoacides selon l'une des revendications 1 et 2 caractérisés en ce que ladite plante oléagineuse est une légumineuse tels le soja, l'arachide ou le lupin, une crucifère tel le colza ou une oléacé.

4. Lipopolyaminoacides selon l'une des revendications 1 à 3 caractérisés en ce que lesdits acides aminés N-acylés sont de formule I suivante : 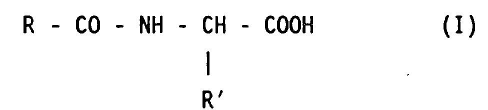 où R représente un radical aliphatique en C₃ - C₂₉, de préférence en C₇ - C₁₉, saturé ou insaturé, linéaire ou ramifié, et R' représente la chaine principale d'un acide aminé issu de l'hydrolyse totale d'une protéine issue d'un tourteau d'une plante oléagineuse.

5. Lipopolyaminoacides selon l'une des revendications 1 à 4 caractérisés en ce que le radical acyle est choisi parmi les radicaux octanoyle, undécylénoyle, lauroyle, cocoyle, palmitoyle, linoléoyle, stéaroyle ou oléoyle.

6. Lipopolyaminoacides selon l'une des revendications 1 à 5 caractérisés en ce qu'ils sont salifiés par l'ammoniaque, les cations des métaux alcalins ou alcalino-terreux, du zinc, du coblat, du fer, du manganese, du cuivre, de l'aluminium ou par des bases organiques telles la monoéthanolamine, la diéthanolamine, la triéthanolamine, la lysine, l'argine, l'histiodine, l'ornithine, la choline ou la morpholine.

7. Lipopolyaminoacides selon l'une des revendications 1 à 6 caractérisés en ce que lesdits acides aminés sont sous forme d'un sel monobasique ou dibasique d'aluminium.

8. Procédé de préparation d'un lipopolyaminoacide selon l'une des revendications 1 à 7 caractérisé en ce qu'on hydrolyse totalement une protéine issue d'un tourteau d'une plante oléagineuse en milieu acide de sorte à obtenir un mélange d'acides aminés, on N-acyle lesdits acides aminés, et le cas échéant, on fait réagir les acides aminés N-acylés avec une base minérale ou organique ou un dérivé métallique en vue de former un sel correspondant.

9. Composition cosmétique caractérisée en ce qu'elle contient des lipopolyaminoacides selon l'une des revendications 1 à 7.

10. Composition alimentaire caractérisée en ce qu'elle contient des lipopolyaminoacides selon l'une des revendications 1 à 7.

11. Composition cosmétique ou alimentaire selon la revendication 9 ou 10 caractérisée en ce qu'elle contient de 0,5 à 20 % en poids, de préférence de 1 à 10% en poids de lipopolyaminoacides selon l'une des revendications 1 à 7.

12. Composition détergente caractérisée en ce qu'elle contient des lipopolyaminoacides selon l'une des revendications 1 à 7.

13. Composition destinée à l'agriculture caractérisée en ce qu'elle contient des lipolyaminoacides selon l'une des revendications 1 à 7, notamment en tant qu'agent de protection contre les parasites, les bactéries et les champignons et/ou agent de stimulation de la croissance des plantes.

14. Médicament caractérisé en ce qu'il contient des lipopolyaminoacides selon l'une des revendications 1 à 7.

15. Utilisation des lipopolyaminoacides selon l'une des revendications 1 à 7 pour l'obtention d'un médicament à activité anti-inflammatoire.

16. Utilisation des lipopolyaminoacides selon l'une des revendications 1 à 7 pour l'obtention d'un médicament à activité antimicrobienne, notamment à activité antibactérienne ou antifongique.

17. Utilisation de lipopolyaminoacides selon l'une des revendications 1 à 7 comme tensioactif.

18. Utilisation cosmétique des lipopolyaminoacides selon la revendication 7 en tant qu'agent antisudoral.

19. Utilisation cosmétique des lipopolyaminoacides selon l'une des revendications 1 à 7 en tant qu'agents anti-radicalaires.

## Patentansprüche

1. Lipopolyaminosäuren, bestehend aus einem durch vollständige Hydrolyse eines Proteins erhaltenen Aminosäuregemisch, in dem die Aminosäuren durch einen C₄-C₃₀-Acylradikal N-acyliert sind, dadurch gekennzeichnet, daß das Protein aus einem Kuchen einer ölhaltigen Pflanze gewonnen wird.

2. Lipopolyaminosäuren nach Anspruch 1, dadurch gekennzeichnet, daß das aus dem Kuchen gewonnene Protein in Form eines Isolats, eines Konzentrats oder eines gereinigten Proteins vorliegt.

3. Lipopolyaminosäuren nach einem der Ansprüche 1 oder 2, dadurch gekennzeichnet, daß die ölhaltige Pflanze eine Hülsenfrucht wie beispielsweise die Sojabohne, die Erdnuß oder die Lupine, eine Kruzifere wie beispielsweise der Raps, oder eine Oleazee ist.

4. Lipopolyaminosäuren nach einem der Ansprüche 1 bis 3, dadurch gekennzeichnet, daß die N-acylierten Aminosäuren die nachfolgende Formeldarstellung I besitzen: 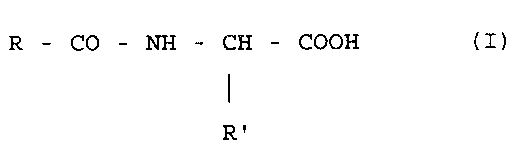 in der R einen aliphatischen, gesättigten oder ungesättigten und linearen oder verzweigten C₃-C₂₉-Radikal, bevorzugt einen C₇-C₁₉-Radikal, und R' die Hauptkette einer aus der vollständigen Hydrolyse eines aus dem Kuchen einer ölhaltigen Pflanze gewonnenen Proteins erhaltenen Aminosäure darstellen.

5. Lipopolyaminosäuren nach einem der Ansprüche 1 bis 4, dadurch gekennzeichnet, daß der Acylradikal aus den Oktanoyl-, Undecylenoyl-, Lauroyl-, Kokoyl-, Palmitoyl-, Linoleoyl-, Stearoyl- oder Oleoyl-Radikalen ausgewählt wird.

6. Lipopolyaminosäuren nach einem der Ansprüche 1 bis 5, dadurch gekennzeichnet, daß diese durch Ammoniak, durch Kationen der alkalischen oder erdalkalischen Metalle, des Zinks, des Kobalts, des Eisens, des Mangans, des Kupfers, des Aluminiums, oder durch organische Basen wie beispielsweise Monoethanolamin, Diethanolamin, Triethanolamin, Lysin, Argin, Histiodin, Ornithin, Cholin oder Morpholin in Salze umgewandelt werden.

7. Lipopolyaminosäuren nach einem der Ansprüche 1 bis 6, dadurch gekennzeichnet, daß die Aminosäuren in Form eines monobasischen oder dibasischen Aluminiumsalzes vorliegen.

8. Verfahren zur Herstellung einer Lipopolyaminosäure nach einem der Ansprüche 1 bis 7, dadurch gekennzeichnet, daß ein aus einem Kuchen einer ölhaltigen Pflanze gewonnenes Protein zum Erhalt eines Aminosäuregemischs in saurer Umgebung vollständig hydrolysiert wird, daß die Aminosäuren N-acyliert werden, und daß die N-acylierten Aminosäuren gegebenenfalls mit einer mineralischen oder organischen Base oder einem Metallderivat zur Reaktion gebracht werden, um ein entsprechendes Salz zu bilden.

9. Kosmetische Zusammensetzung, dadurch gekennzeichnet, daß sie Lipopolyaminosäuren nach einem der Ansprüche 1 bis 7 enthält.

10. Nährstoff-Zusammensetzung, dadurch gekennzeichnet, daß sie Lipopolyaminosäuren nach einem der Ansprüche 1 bis 7 enthält.

11. Nährstoff- oder kosmetische Zusammensetzung nach Anspruch 9 oder 10, dadurch gekennzeichnet, daß sie zwischen 0,5 und 20 Gewichtsprozent, bevorzugt zwischen 1 und 10 Gewichtsprozent, an Lipopolyaminosäuren nach einem der Ansprüche 1 bis 7 enthält.

12. Waschmittel-Zusammensetzung, dadurch gekennzeichnet, daß sie Lipopolyaminosäuren nach einem der Ansprüche 1 bis 7 enthält.

13. Für die Landwirtschaft bestimmte Zusammensetzung, dadurch gekennzeichnet, daß sie Lipopolyaminosäuren nach einem der Ansprüche 1 bis 7 enthält, insbesondere als Mittel zum Schutz gegen Parasiten, Bakterien und Pilze und/oder als Mittel zur Stimulierung des Pflanzenwachstums.

14. Medikament, dadurch gekennzeichnet, daß es Lipopolyaminosäuren nach einem der Ansprüche 1 bis 7 enthält.

15. Verwendung der Lipopolyaminosäuren nach einem der Ansprüche 1 bis 7 zur Herstellung eines Medikaments mit entzündungshemmender Wirkung.

16. Verwendung der Lipopolyaminosäuren nach einem der Ansprüche 1 bis 7 zur Herstellung eines Medikaments mit antimikrobieller Wirkung, insbesondere mit antibakterieller und antifungizider Wirkung.

17. Verwendung von Lipopolyaminosäuren nach einem der Ansprüche 1 bis 7 als Tensid.

18. Kosmetische Verwendung der Lipopolyaminosäuren nach einem der Ansprüche 1 bis 7 als schweißhemmendes Mittel.

19. Kosmetische Verwendung der Lipopolyaminosäuren nach einem der Ansprüche 1 bis 7 als gegen Radikale wirkendes Mittel.

## Claims

1. Lipopolyamino acids consisting of a mixture of amino acids obtained by total hydrolysis of a protein, the said amino acids being N-acylated by an acyl radical with C₄ - C₃₀, characterized in that the protein is derived from a cake of an oleaginous plant.

2. Lipopolyamino acids according to claim 1 characterized in that the said protein derived from a cake is in the form of an isolate, a concentrate or a purified protein.

3. Lipopolyamino acids according to either of claims 1 or 2 characterized in that the said oleaginous plant is a leguminous plant such as soya, ground-nut or lupin, a cruciferous plant such as rape or an oleaceous plant.

4. Lipopolyamino acids according to one of claims 1 to 3 characterized in that the said N-acylated amino acids have the following formula I: 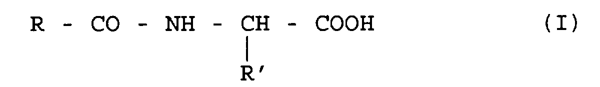 where R represents an aliphatic saturated or unsaturated, linear or branched radical with C₃ - C₂₉, preferably with C₇ - C₁₉, and R' represents the main chain of an amino acid resulting from the total hydrolysis of a protein derived from a cake of an oleaginous plant.

5. Lipopolyamino acids according to one of claims 1 to 4 characterized in that the acyl radical is selected from octanoyl, undecylenoyl, lauroyl, cocoyl, palmitoyl, linoleoyl, stearoyl or oleoyl radicals.

6. Lipopolyamino acids according to one of claims 1 to 5 characterized in that salts of these are prepared with ammonia, cations of alkali metals or alkaline earths, zinc, cobalt, iron, manganese, copper, aluminium or with organic bases such as monoethanolamine, diethanolamine, triethanolamine, lysine, arginine, histidine, ornithine, choline or morpholine.

7. Lipopolyamino acids according to one of claims 1 to 6 characterized in that the said amino acids are in the form of a monobasic or dibasic salt of aluminium.

8. Process for the preparation of a lipopolyamino acid according to one of claims 1 to 7 characterized in that a protein derived from a cake of an oleaginous plant is totally hydrolysed in an acid medium so as to obtain a mixture of amino acids, the said amino acids are N-acylated, and where appropriate, the N-acylated amino acids are reacted with an inorganic or organic base or a metallic derivative with a view to forming a corresponding salt.

9. Cosmetic composition characterized in that it contains lipopolyamino acids according to one of claims 1 to 7.

10. Nutritive composition characterized in that it contains lipopolyamino acids according to one of claims 1 to 7.

11. Cosmetic or nutritive composition according to claim 9 or 10, characterized in that it contains 0.5 to 20 % by weight, preferably 1 to 10 % by weight, of lipopolyamino acids according to one of claims 1 to 7.

12. Detergent composition characterized in that it contains lipopolyamino acids according to one of claims 1 to 7.

13. Composition intended for agriculture, characterized in that it contains lipopolyamino acids according to one of claims 1 to 7, in particular as a protective agent against parasites, bacteria and fungi and/or an agent stimulating the growth of plants.

14. Medication characterized in that it contains lipopolyamino acids according to one of claims 1 to 7.

15. Use of lipopolyamino acids according to one of claims 1 to 7 for obtaining a medication with anti-inflammatory activity.

16. Use of lipopolyamino acids according to one of claims 1 to 7 for obtaining a medication with anti-microbial activity, particularly antibacterial or antifungal activity.

17. Use of lipopolyamino acids according to one of claims 1 to 7 as a surface active agent.

18. Cosmetic use of lipopolyamino acids according to claim 7 as an antisudoral agent.

19. Cosmetic use of lipopolyamino acids according to one of claims 1 to 7 as anti-radical agents.
